# FASCICULE DE BREVET EUROPEEN

(11) **EP 1 263 978 B1**
(45) Date de publication et mention de la délivrance du brevet: **07.12.2005**
(21) Numéro de dépôt: 01914000.3
(22) Date de dépôt: 14.03.2001
(51) Int. Cl.: C12N 15/85, C12N 15/63, C12N 9/20, C07K 14/705, C07K 14/78

(54) **PROMOTEURS HYBRIDES INDUCTIBLES PAR L'INFLAMMATION, VECTEURS LES CONTENANT ET UTILISATIONS**
DURCH ENTZÜNDUNGSVERMITTLER INDUZIERBARE HYBRIDPROMOTOREN, DIESE ENTHALTENDE VEKTOREN UND VERWENDUNGEN DAVON
INFLAMMATION-INDUCIBLE HYBRID PROMOTERS, VECTORS CONTAINING SAME AND USES THEREOF

(30) Priorité: 14.03.2000 FR 0003262; 13.04.2000 US 196959 P
(43) Date de publication de la demande: 11.12.2002
(73) Titulaire: UNIVERSITE PIERRE ET MARIE CURIE, 75252 Paris Cédex 05 (FR)
(72) Inventeur: MASSAAD, Charbel, F-92140 Clamart (FR); BERENBAUM, Françis, F-91190 Gif Sur Yvette (FR); OLIVIER, Jean-Luc, F-54000 Nancy (FR); SALVAT, Colette, F-75011 Paris (FR); BEREZIAT, Gilbert, F-91120 Palaiseau (FR)
(74) Mandataire: Desaix, Anne
(86) Numéro de dépôt international: PCT/FR2001/000759
(87) Numéro de publication internationale: WO 2001/068845

(56) Documents cités:
- WO-A-00/78986
- WO-A-98/21349
- WO-A-98/57631
- GB-A- 2 269 897
- COUTURIER C ET AL: "Interleukin 1beta induces type II-secreted phospholipase A(2) gene in vascular smooth muscle cells by a nuclear factor kappaB and peroxisome proliferator-activated receptor-mediated process" JOURNAL OF BIOLOGICAL CHEMISTRY., vol. 274, no. 33, 13 août 1999 (1999-08-13), pages 23085-23093, XP002154468
- JUGE-AUBRY C ET AL: "DNA binding properties of peroxisome proliferator-activated receptor subtypes on various natural peroxisome proliferator response elements. Importance of the 5'-flanking region" JOURNAL OF BIOLOGICAL CHEMISTRY., vol. 272, no. 40, 3 octobre 1997 (1997-10-03), pages 25252-25259, XP002154469 cité dans la demande
- MUKHOPADHYAY K ET AL: "Use of a new rat chondrosarcoma cell line to delineate a 119-base pair chondrocyte-specific enhancer element and to define active promoter segments in the mouse pro-alpha 1(II) collagen gene" JOURNAL OF BIOLOGICAL CHEMISTRY., vol. 270, no. 46, 17 novembre 1995 (1995-11-17), pages 27711-27719, XP002154470 cité dans la demande
- MASSAAD C ET AL: "Induction of secreted type IIA phospholipase A2 gene transcription by interleukin-1beta. Role of C/EBP factors" JOURNAL OF BIOLOGICAL CHEMISTRY., vol. 275, no. 30, 28 juillet 2000 (2000-07-28), pages 22686-22694, XP002154471
- BORDJI K ET AL: "Evidence for the presence of peroxisome proliferator-activated receptor (PPAR) alpha and gamma and retinoid Z receptor in cartilage. PPARgamma activation modulates the effects of interleukin-1beta on rat chondrocytes" JOURNAL OF BIOLOGICAL CHEMISTRY., vol. 275, no. 16, 21 avril 2000 (2000-04-21), pages 12243-12250, XP002176055 cité dans la demande

## Description

### DOMAINE TECHNIQUE

La présente invention concerne le domaine de la biologie. Elle concerne notamment le domaine de la régulation de l'expression de gènes et, plus particulièrement, elle décrit la mise au point et le développement d'un nouveau système de régulation pharmacologique de l'expression de transgènes. L'invention repose notamment sur l'utilisation de constructions permettant d'activer la transcription du transgène dans des cellules, tissus ou organes en condition d'inflammation. L'invention décrit ainsi de nouvelles compositions, constructions pour la régulation efficace de l'expression d'un acide nucléique in vitro, ex vivo ou in vivo, par exemple dans les cellules chondrocytaires. Les applications qui découlent de la présente invention sont nombreuses, dans les domaines expérimentaux, cliniques, thérapeutiques ou diagnostiques, par exemple.

### ETAT DE LA TECHNIQUE ANTERIEURE

Le contrôle du niveau et de la durée de l'expression des transgènes est nécessaire pour de nombreuses applications. Ainsi, la thérapie génique, la production de protéines recombinantes in vitro, la construction d'animaux transgéniques, l'étude des effets d'un gène ou de la biodisponibilité d'une protéine, etc. sont autant de situations dans lesquelles un contrôle approprié de l'expression génétique peut être mis en oeuvre et apporter des améliorations.

La thérapie génique vise plus particulièrement à exprimer des gènes (ou acides nucléiques) soit défectifs chez un individu donné, soit d'intérêt thérapeutique dans le cadre d'une pathologie spécifique. L'expression de ces gènes nécessite, en dehors de procédure de vectorisation, une induction par un promoteur qui fixe l'ARN polymérase et les facteurs de transcription requis pour obtenir la synthèse d'ARN messager et donc la synthèse protéique. Les promoteurs viraux ou eucaryotes non-inductibles sont fréquemment utilisés en thérapie génique pour exprimer un gène au niveau d'un tissu. Bien que facile à réaliser, cette approche ne permet pas la régulation de l'expression du transgène en fonction de l'environnement et des stimuli externes. De plus, le gène dirigé par un promoteur viral s'exprimera indifféremment dans toutes les cellules dans lesquelles le vecteur aura pénétré, sans aucun ciblage. Enfin, les expérimentateurs se sont parfois heurtés à la faiblesse de l'expression du promoteur viral *in vivo* par rapport à celle constatée *in vitro.*

Différents régulateurs de transcription artificiels ont été conçus dans l'art antérieur, activés par une molécule xénobiotique qui se lient sur les séquences promotrices de la transcription du transgène.

Une première illustration de ces régulateurs a été construite par fusion du répresseur Lac de E. coli avec le domaine transactivateur de VP16 du virus herpès simplex (HSV). Deux versions de ces régulateurs existent, l'une pouvant être activée par l'isopropyl b-D-thiogalactoside (IPTG) et l'autre inactivée par l'IPTG (Baim S. et coll., *Proc Natl Acad Sci U S A,* 88 (1991) 5072-5076 ; Labow M. et coll., *Mol. Cell. Biol.,* 10 (1990) 3343-3356).

Un autre système a été construit par fusion du répresseur Tet de E. coli avec le domaine transactivateur de VP16 de HSV. Il existe également deux versions de ces régulateurs, l'une pouvant être activée par la tétracycline ou ses dérivés et l'autre inactivée par ces mêmes molécules (Gossen M. et Bujard H., *Proc Natl Acad Sci U S A,* 89 (1992) 5547-5551; Gossen M. et coll., *Science,* 268 (1995) 1766-1769).

Un autre système a été construit par fusion du domaine de liaison à l'ADN de la protéine GAL4 de S. cerevisiae avec le domaine de liaison au ligand du récepteur humain à la progestérone et le domaine transactivateur de VP16 de HSV, cette version est activée par un analogue de la progestérone tel que le RU486 (Wang Y. et coll., *Proc Natl Acad Sci U S A,* 91 (1994) 8180-8184). Une fusion du récepteur à l'ecdysone de drosophile avec le domaine transactivateur de VP16 de HSV a également été décrite, activée par l'ecdysone et les analogues de cette hormone stéroïdienne (No D. et coll., *Proc Natl Acad Sci U S A*, 93 (1996) 3346-3351). Un autre système tire parti de la capacité de certaines molécules immunosuppressives (cyclosporine A, rapamycine et ses dérivés) de promouvoir l'association de certaines protéines cellulaires. Un régulateur transcriptionnel est alors constitué de deux sous-unités protéiques, la première peut être formée par la fusion d'un domaine de liaison à l'ADN chimérique et de trois copies de la protéine humaine FKBP et la deuxième par la fusion du domaine de liaison à la rapamycine de la protéine humaine FRAP et du domaine transactivateur de la sous-unité p65 de NFkB humain. Ce régulateur transcriptionnel est activé par la rapamycine qui permet la dimérisation des deux sous-unités (Rivera V. et coll., *Nat. Med.,* 2 (1996) 1028-1032).

Couturier et al, JBC vol. 274 n° 33 (1999) décrit que le promoteur du rat type II-s PLA2 est homologue à l'élément DR 1 du site de liaision PPAR. WO 9821349 décrit une cassette d'expression contenant un élément de réponse à un PPAR et le promoteur du gène humain de l'apolipoprotéine AII. Cependant ces références n'enseignent pas l'obtention d'un promoteur humain PLA 2 à partir d'une construction de PPAR en amont de celui-ci.

Même si ces systèmes permettent d'obtenir des niveaux de régulation satisfaisants dans certains tissus, ils présentent néanmoins certains inconvénients qui limitent leurs conditions d'utilisation. Ainsi, ces régulateurs transcriptionnels sont des protéines xenogéniques chez l'homme. Elles sont en effet constituées de fragments protéiques provenant de bactérie, de virus, de levure ou d'insecte ou, lorsque les domaines protéiques sont d'origine humaine, leur jonction crée des séquences étrangères à l'homme. Ces domaines protéiques peuvent donc induire une réaction immunitaire cytotoxique, entraînant la destruction des cellules qui expriment le gène d'intérêt sous contrôle du régulateur transcriptionnel xénogénique, et ainsi l'arrêt de l'expression du transgène. Cette situation peut imposer le recours à des administrations répétées du gène thérapeutique, ce qui constitue un inconvénient important, notamment lorsque cela implique un acte chirurgical traumatique, et qui n'est pas toujours efficace, notamment lorsque le vecteur du gène thérapeutique est un virus dont la première injection provoque une réaction immunitaire. En outre, les niveaux d'expression observés avec les systèmes de régulation de l'art antérieur ne sont pas toujours satisfaisants.

La présente invention décrit maintenant un nouveau système amélioré pour l'expression contrôlée d'acides nucléiques dans des cellules. Cette invention propose un promoteur hybride inductible par les principaux médiateurs de l'inflammation et par des composés exogènes. Ce promoteur conduit préférentiellement l'expression d'un gène au niveau du tissu siège d'une inflammation, en réponse aux médiateurs synthétisés au cours du processus inflammatoire. L'intérêt principal de ce promoteur réside dans la possibilité d'une expression du gène-médicament modulable en fonction de l'intensité de l'inflammation. De plus, l'expression du gène peut-être augmentée en cas de prise d'AINS. Il s'agit donc d'une approche transposable à de nombreuses situations inflammatoires pour lesquelles une thérapie génique est envisagée.

La présente invention réside plus particulièrement dans la construction d'un promoteur hybride comprenant un module de réponse aux PPAR et un module de réponse aux cytokines produites lors de l'inflammation. Ce deuxième module comprend plus particulièrement des séquences dérivées du promoteur du gène de la phospholipase A2 sécrétée non pancréatique (PLA2s) de type IIA-1, qui est exprimé dans certaines cellules humaines, notamment les chondrocytes, dans des conditions d'inflammation. La combinaison de ces deux modules permet de générer un promoteur assurant une expression spécifique de gènes en condition d'inflammation, expression qui peut être augmentée par les médiateurs ou des médicaments anti-inflammatoires exogènes. Comme il sera illustré dans les exemples, ce promoteur hybride possède préférentiellement une activité basale faible, mais une activité en condition d'induction très élevée. Ce type de promoteur est donc particulièrement adapté à l'expression de produits ayant une activité anti-inflammatoire, comme il sera expliqué en détails dans la suite du texte. Les promoteurs de l'invention sont tout particulièrement avantageux pour l'expression de gènes dans les tissus articulaires, notamment les chondrocytes, dans différentes situations pathologiques dans lesquelles une composant inflammatoire est présente, et en particulier l'arthrose.

L'arthrose est une maladie du vieillissement qui s'accompagne de destructions articulaires entraînant un degré variable d'invalidité (Chevalier X, 1998). Le traitement de cette pathologie est actuellement considéré comme une priorité en terme de santé publique du fait de sa fréquence et du handicap qu'elle provoque. Au plan physiopathologique, même si les mécanismes d'initiation restent encore mal compris, la cible tissulaire responsable de la destruction articulaire est le cartilage, tissu composé d'une matrice extracellulaire riche en collagène de type II et en protéoglycanes et des chondrocytes articulaires, seul type cellulaire du cartilage, qui sont responsables de la synthèse matricielle. Les chondrocytes sécrètent également des facteurs de croissance et des inhibiteurs naturels de protéases (activité anabolique) ainsi que des protéases (activité catabolique). Physiologiquement, il existe un équilibre entre ces activités (homéostasie cartilagineuse). Au cours de l'arthrose, on assiste à un déséquilibre au détriment de l'activité anabolique du fait de la stimulation de synthèse de protéases (essentiellement des métalloprotéases) et d'une inhibition de synthèse d'inhibiteurs de métalloprotéases (TIMP ou tissu inhibitor of metalloproteases) (Lefebvre V et al, 1997; Dean J et al, 1990; Hornebeck W, 1990). Ces modifications de synthèse sont dues à la présence de médiateurs pro-inflammatoires produits par les chondrocytes et retrouvés en grandes quantités dans les liquides synoviaux de malades souffrant d'arthrose ou de polyarthrite rhumatoïde, des cytokines (IL-1, TNF) et des médiateurs lipidiques, en particulier la prostaglandine E2. Cette prostaglandine, stimule la synthèse de collagène à basse concentration comme le facteur de croissance mais l'inhibe à haute concentration (Di Battista JA et al, 1996).

La présente invention propose à présent des promoteurs transcriptionnels hybrides qui sont particulièrement adaptés à l'expression de gènes thérapeutiques dans les pathologies articulaires comme l'arthrose. En outre, l'invention décrit également des constructions assurant un ciblage du transfert et/ou de l'expression d'un gène d'intérêt dans les chondrocytes, améliorant encore les propriétés avantageuses des constructions pour ce type d'applications.

L'invention réside plus particulièrement dans un promoteur hybride comprenant :
a) un élément de réponse à un PPAR comprenant un ou plusieurs sites de liaison du PPAR et
b) tout du promoteur du gène Humain de la phospholipase A2 sécrétée non-pancréatique PLA2S ou partie de celui-ci assurant une activité transcriptionnelle basale au promoteur hybride et un caractère régulable par les médiateurs de l'inflammation, dans lequel l'élément (a) est positionné en amont de l'élément (b).

La présente invention réside notamment dans la mise en évidence que ce type de construction produit un effet synergique sur l'activité d'expression, sans altérer le caractère régulé et spécifique (ou préférentiel) des situations d'inflammation. L'invention décrit également des vecteurs, plasmides et compositions comprenant ces promoteurs, pour l'expression de gènes in vitro, ex vivo ou in vivo.

Le premier élément des promoteurs hybrides de l'invention comprend donc un élément de réponse à un PPAR. Les PPARs (Peroxisome Proliferator-Activated Receptors) appartiennent à la superfamille des récepteurs nucléaires comprenant les récepteurs aux hormones stéroïdes, les récepteurs aux hormones thyroidiennes à l'acide rétinoïque et à la vitamine D (Schoonjans K et al, 1996). Les PPARs existent sous trois isoformes : le PPARα, le PPARβ et le PPARγ. Les PPAR se lient à des séquences spécifiques au niveau des promoteurs de gènes cibles (séquences PPRE), et induisent l'expression de ces gènes.

Un élément de réponse à un PPAR (PPRE, "Peroxysome Proliferator Response Element") est donc une région d'acide nucléique capable de fixer un PPAR, la liaison du PPAR pouvant ensuite médier un signal vers des régions nucléiques voisines, notamment les régions dérivées de PLA2s selon l'invention. Un élément de réponse à un PPAR selon l'invention est plus particulièrement une région d'acide nucléique capable de lier un PPAR, qu'il s'agisse de PPARα, β ou γ. Il s'agit encore plus préférentiellement d'un acide nucléique capable de lier un PPARα ou un PPARγ. Pour la mise en oeuvre de l'invention, l'élément de réponse à un PPAR comprend plus particulièrement un ou plusieurs sites de liaison du PPAR. De tels sites de liaison ont été décrits dans l'art antérieur, comme par exemple dans différents promoteurs humains (site consensus DR1, gène de l'apolipoprotéine All, par exemple). De tels sites peuvent également être construits artificiellement, et testés pour leurs propriétés de PPRE, comme il est décrit ci-après.

Dans un mode particulier de réalisation de l'invention, l'élément de réponse à un PPAR comprend un ou plusieurs sites de séquence CAAAACTAGGTCAAAGGTCA (SEO ID NO:1) ou de variants fonctionnels de cette séquence. La séquence SEQ ID NO:1 correspond à la région consensus DR1.

Le terme variant fonctionnel désigne toute séquence modifiée conservant les propriétés de PPRE telles que mentionnées ci-dessus, c'est-à-dire notamment la capacité de lier un PPAR. Les modifications peuvent comprendre une ou plusieurs additions, mutations, délétions et/ou substitutions de nucléotides dans la séquence considérée. Ces modifications peuvent être introduites par les méthodes classiques de la biologie moléculaire, telles que notamment la mutagénèse dirigée ou, plus pratiquement, par synthèse artificielle de la séquence dans un synthétiseur. Généralement, les variants conservent au moins 50% des résidus de la séquence initiale indiquée. Plus préférentiellement, les variants possèdent des modifications affectant moins de 8 nucléotides dans la séquence considérée. Les variants ainsi obtenus sont ensuite testés pour leur activité de PPRE. Cette propriété peut être vérifiée de différentes façons, et notamment:
(i) par mise en contact de la séquence test avec un PPAR (par exemple dans un test acellulaire), et détection de la formation d'un complexe (par exemple par retard de migration sur gel);
(ii) par insertion de la séquence test dans une cassette d'expression comprenant un promoteur minimal et un gène rapporteur, introduction de la cassette dans une cellule, et détection (le cas échéant dosage) de l'expression du gène rapporteur en présence et en l'absence d'un PPAR et d'un ligand d'un PPAR;
(iii) par toute autre technique connue de l'homme du métier, permettant de mettre en évidence l'interaction entre un acide nucléique et une protéine, par exemple.

Un variant est considéré comme fonctionnel au sens de la présente invention lorsque l'activité mesurée, par exemple en (ii) ci-dessus, est préférentiellement au moins égale à 50% de celle mesurée avec un site de séquence SEQ ID N0:1, plus préférentiellement au moins égale à 75%. Des variants fonctionnels de sites de liaison de PPAR au sens de l'invention sont décrits par exemple dans Juge-Aubry et al. (J. Biol. Chem. 272 (1997) 25252) et dans Nakshatri et al. (NAR 26 (1998) 2491).

Des exemples spécifiques d'éléments de réponse à un PPAR selon l'invention sont représentés par les éléments (DR1)2 17 (SEQ ID NO :2), (DR1)2 21 (SEQ ID NO:3) et (DR1)2 31 (SEQ ID NO:4) tels que décrits dans les exemples, ou des variants fonctionnels de ceux-ci.

Un autre variant est un fragment de la séquence SEQ ID NO:1, comprenant les résidus 8-20 de celle-ci.

Les séquences SEQ ID NO :1-4 selon l'invention représentent des PPRE répondant essentiellement aux PPARα et γ. Comme il est illustré dans les exemples, ces séquences possèdent une forte inductibilité par les PPAR, liée notamment à l'agencement particulier des sites de liaison. A cet égard, un objet particulier de l'invention réside également dans un acide nucléique comprenant une séquence choisie parmi SEQ ID NOS :2-4.

Selon un autre mode particulier de réalisation, l'élément de réponse à un PPAR comprend un ou plusieurs sites J du promoteur de l'apoAll humain (nucléotides -734 à -716) ou des variants fonctionnels de cette séquence, qui répond à l'activation par les PPARβ.

Comme indiqué ci-avant, dans les compositions selon l'invention, l'élément de réponse au PPAR peut comprendre plusieurs sites de liaison à un PPAR. Il peut s'agir d'une répétition d'un même site, ou de combinaisons de sites différents, la répétition de sites identiques étant préférée. Plus particulièrement, l'élément de réponse comprend jusqu'à 30 sites de liaison, de préférence de 3 à 20, plus préférentiellement de 1 à 5. Un mode de réalisation préféré de l'invention est une construction comprenant 2 sites de liaison DR1, les résultats présentés dans les exemples montrant les propriétés avantageuses de telles constructions en termes d'induction et de niveaux d'expression, notamment dans les cellules chondrocytes (SEQ ID NO : 2-4).

Pour la réalisation d'un promoteur hybride selon l'invention, l'élément (a) de réponse à un PPAR est associé à tout ou une partie du promoteur PLA2s IIA-1 (élément b)).

La phospholipase A2 sécrétée non pancréatique est une protéine activée par l'interleukine 1 bêta, et conduit à la production de médiateurs de l'inflammation, notamment de médiateurs lipidiques (prostaglandine E2). En particulier, la prostaglandine PGE2 est issue de l'activation par l'interleukine-1 (IL-1) de la phospholipase A₂ sécrétée non pancréatique (PLA₂s) (Nevalainen TJ et al, 1993; Hulkower KJ, 1997) qui libère l'acide arachidonique des phospholipides et de la cyclooxygènase inductible (COX-2) (Amin AR, 1997; Geng Y et al 1995; Knott et al 1994), cette dernière convertit l'acide arachidonique en PGH2. La PGH2 est ensuite convertie en PGE2 par la PGE synthase. Deux types de phospholipases A₂ ont été mis en évidence dans les cellules humaines, notamment dans les chondrocytes. La phospholipase A₂ sécrétée non pancréatique (PLA₂s) est une protéine de 14 kDa abondamment synthétisée et sécrétée dans les liquides synoviaux inflammatoires (Seilhamer JJ et al , 1989). La phospholipase A₂ cytosolique (PLA₂c) est une protéine cytosolique de 85 Kda dont l'activation passe par sa phosphorylation par les MAP kinases et sa translocation à la membrane (Clark JD et al, 1991). L'IL-1 induit l'expression des gènes COX-2 et PLA₂s sans affecter celle du gène PLA₂c dans les chondrocytes (Berenbaum F et al, 1994; Jacques C et al, 1997). Les taux de PLA₂ sécrétée dans l'articulation et dans le sérum sont corrélés avec le degré de l'inflammation, notamment le degré de l'arthrose dans le cas des chondrocytes (Pruzanski W et al, 1994; Lin M et al, 1996). La coopération entre la PLA2s et la COX-2 aboutit à une production prolongée (>48 heures) de prostaglandine PGE2 sous la stimulation de l'lL-1β (Bingham CO et al 1996; Kuwata H et al, 1998) tandis que la PLA2c et la cyclooxygénase constitutive COX-1 seraient responsables d'une réponse précoce. Dans nos travaux précédents, une corrélation entre la synthèse de PGE2 et le taux d'activité de PLA₂s liée aux membranes chondrocytaires avait été montrée (Jacques C et al 1997).

La présente invention propose à présent des compositions comprenant tout ou partie du promoteur du gène PLA2s pour réguler l'expression d'acides nucléiques d'intérêt, dans des compositions thérapeutiques notamment. L'invention propose notamment des compositions comprenant tout ou des parties fonctionnelles du promoteur PLA2s IIA, pour la construction d'un promoteur hybride régulé actif dans les cellules présentant une situation d'inflammation. A cet égard, les demandeurs ont montré qu'un fragment de 247 paires de base en amont du premier exon du gène humain de la PLA2s IIA (SEQ ID NO :5) était responsable d'une stimulation de 6 à 8 fois de l'activité du gène rapporteur CAT par l'IL1β dans les chondrocytes de lapin en culture primaire. Trois régions régulatrices ont été caractérisées dans ce promoteur : La région proximale (-85/-114) assure une activité transcriptionnelle basale et lie le facteur Sp1 ; La région distale (-247/-210) lie des membres de la famille NF1 et le récepteur aux glucocorticoïdes, qui stabilisent la fixation des facteurs C/EBP entre les positions - 198/-190 par rapport au site d'initiation de la transcription. La famille des facteurs C/EBP comporte trois membres activateurs principaux C/EBPα, C/EBPβ et C/EBPδ. Les facteurs C/EBPβ et C/EBPδ sont activés au niveau transcriptionnel et post-transcriptionnel par les cytokines pro-inflammatoires. Ils sont exprimés dans les chondrocytes alors que C/EBP_{α} ne l'est pas. C/EBP_{β} et C/EBPδ sont responsables de l'activation de la transcription du gène de la sPLA2-IIA par l'IL1β. Les demandeurs ont maintenant montré qu'ils interviennent également de manière critique dans l'induction de la transcription du gène de la COX-2 par l'IL-1β dans les chondrocytes humains et de lapin.

Dans un mode particulier de réalisation, l'élément b) des promoteurs hybrides de l'invention comprend tout ou partie de la séquence SEQ ID N :5 ou d'un dérivé fonctionnel de celle-ci. La séquence SEQ ID NO :5 correspond aux nucléotides -247 à +20 du gène humain de la PLA2s IIA (résidus 5 à 271).

Le terme « partie » désigne plus particulièrement tout fragment de la séquence considérée conservant une fonctionnalité biologique. Il s'agit plus particulièrement d'une partie assurant une activité transcriptionnelle basale au promoteur hybride (boite TATA, par exemple) et un caractère régulable par les médiateurs de l'inflammation, notamment par l'interleukine-1β.

A cet égard, une variante préférée de l'invention concerne un promoteur hybride comprenant au moins une partie du promoteur PLA2s IIA conférant l'induction par l'interleukine-1β.

Plus particulièrement l'élément (b) d'un promoteur hybride selon l'invention peut comprendre les séquences suivantes :
- au moins les résidus 51 à 61 de SEQ ID NO :5 (correspondant environ aux résidus -200/-191 du gène PLA2s IIA humain), impliqués dans la régulation par l'IL1, et/ou,
- au moins les résidus 23 à 32 de SEQ ID NO :5 (correspondant environ aux résidus -229/-220 du gène PLA2s IIA humain), représentant un demi site NF1 et un demi site GRE (ces séquences sont impliquées dans la régulation par certains composés comme la déxamethasone), et/ou
- au moins les résidus 148 à 155 de SEQ ID NO :5 (correspondant environ aux résidus -104/-97 du gène PLA2s IIA humain), impliqués dans la liaison de Sp1, et/ou
- au moins les résidus 5 à 170 de SEQ ID NO :5 (correspondant environ aux résidus -247/-85 du gène PLA2s IIA humain), ou encore
- au moins les résidus 51 à 170 de SEQ ID NO :5 (correspondant environ aux résidus -200/-85 du gène PLA2s IIA humain).

Un exemple spécifique de promoteur hybride comprend un élément de réponse au PPAR (DR1)2 21 lié au fragment -247/+20 du gène PLA2s IIA. Un tel promoteur comporte la séquence SEQ ID NO:6, qui est également un objet particulier de la présente invention. Dans cette séquence, l'élément PPRE correspond essentiellement aux résidus 13-53 et l'élément PLA2s aux résidus 62-332. Les résidus 1-12 et 54-61 correspondent à des régions neutres sur le plan fonctionnel, résultant d'étapes de clonage et permettant de modifier la structure du promoteur (sites de clonage).

Bien entendu, des variations de séquence peuvent intervenir dans les régions considérées, sans affecter de manière substantielle l'activité transcriptionnelle du promoteur hybride de l'invention (mutations, délétions, substitutions, insertions, etc.), notamment sur une à cinq bases. Il est entendu que de tels variants représentent des modes de réalisation spécifiques de la présente invention.

L'élément de réponse au PPAR (PPRE) et la région dérivée de PLA2s IIA sont agencés de manière fonctionnelle dans le promoteur hybride de l'invention, c'est-à-dire de sorte que le promoteur hybride exerce une activité transcriptionnelle en présence de médiateurs de l'inflammation, de préférence régulée par l'élément PPRE.

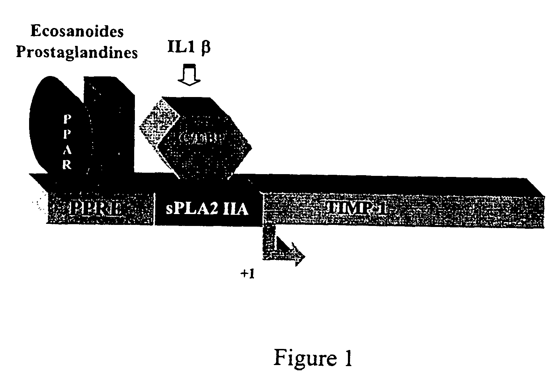

Préférentiellement, l'élément a) est positionné en amont (en 5') de l'élément (b) dans le promoteur hybride (Cf figure 1). Cette configuration permet en effet d'assurer un niveau d'expression élevé et régulé dans les cellules, en présence de médiateurs de l'inflammation (LTB4) et/ou d'activateurs de PPAR.

Les différents domaines fonctionnels ci-dessus peuvent être liés directement les uns aux autres, ou séparés par ou associés à des nucléotides n'affectant pas significativement le caractère régulé du promoteur. De tels nucléotides peuvent être des résidus neutres sur le plan fonctionnel, résultant par exemple d'étapes de clonage (extrémités PCR, sites de restriction, etc.). Ces nucléotides peuvent aussi posséder des propriétés biologiques, permettant de conférer des caractéristiques ou performances améliorées au système de l'invention (amplificateur de gènes de ménage, amplificateur tissus spécifiques, silenceur, intron, site d'épissage, etc.). A cet égard, dans un mode de réalisation particulier de l'invention, le promoteur hybride comprend en outre un élément c) conférant une spécificité tissulaire. Cet élément c) peut être localisé entre les éléments a) et b), ou en aval (3') de l'élément b). Le terme « spécificité tissulaire » désigne une expression préférentielle ou majoritaire dans un (type) de cellules donné, sans toutefois exclure totalement une expression résiduelle ou moins importante dans d'autres cellules.

Un objet particulier de l'invention réside plus spécifiquement dans un promoteur transcriptionnel hybride comprenant, dans l'orientation 5'->3' :
a) un élément de réponse à un PPARα et/ou γ, comprenant de préférence tout ou partie d'une séquence SEQ ID NO :1 à 4,
b) tout ou partie de la séquence SEQ ID NO :5, et
c) le cas échéant, un élément conférant une spécificité tissulaire.

Dans une application particulière, l'élément c) confère une spécificité d'expression pour les cellules chondrocytaires, et notamment il comprend tout ou partie de la séquence SEQ ID NO:7 ou d'un variant de celle-ci. Ce type de promoteur est particulièrement adapté à l'expression régulée et sélective d'acides nucléiques dans les chondrocytes, notamment en situation d'inflammation (arthrose).

La matrice cartilagineuse est constituée d'un grand nombre de protéines collagéniques et non collagéniques. Certains gènes codant pour des protéines de cette matrice sont exprimés spécifiquement par le chondrocyte comme les gènes du collagène de type II, de l'agrécane, de la matrilin-1 (ou cartilage matrix protein) et de la chondroadhérine. Ces deux dernières protéines interagissent spécifiquement avec des intégrines synthétisées par les chondrocytes et sont responsables d'un ancrage de la cellule aux autres composants de la matrice (Makihira S et al, 1999; Camper L et al 1997). L'expression des gènes du collagène de type II et de l'agrécane ont été abondamment cités dans la littérature comme des marqueurs positifs de la différenciation chondrocytaire alors que la synthèse du collagène de type I signe un phénomène de dédifférenciation (Benya P et al, 1986). Le groupe du Pr de Crombrugghe a identifié une séquence de 18 paires de bases nécessaire et suffisante pour diriger l'expression du collagène de type II dans les chondrocytes. Cette séquence fixe un facteur nucléaire (SOX9) qui serait par ailleurs impliqué dans la différenciation chondrocytaire (Mukhopadhyay K et al, 1995; Zhou G et al, 1995). Ce facteur est colocalisé avec l'expression du Collagène de type II dans les chondrocytes primaires. Il se lie à la séquence CATTCAT au niveau du promoteur du gène de collagène de type II et active sa transcription.

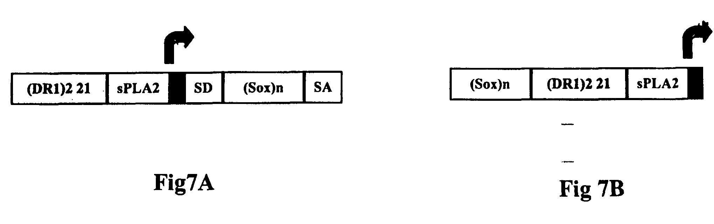

Pour que le gène d'intérêt soit exprimé non plus seulement de manière inductible mais aussi spécifiquement dans le chondrocyte, des éléments du promoteur du collagène de type II capables de fixer les facteurs SOX (SOX9, SOX6 et L-SOX5) (Mukhopadhyay K et al, 1995; Zhou G et al, 1995; Lefebvre V et al 1997) peuvent être intégrés. Une séquence nucléotidique décrite par Zhou et al (1995) et Lefebvre V et al (1997) formée d'un duplicata de 231 pb du premier intron du collagène de type Il peut être utilisée. Cette construction s'est avérée être la plus efficace dans le système. Elle peut être insérée en aval de la construction hybride (DR1)2 21 -promoteur de la sPLA2 IIA, en incluant des sites d'épissage donneur (SD) et accepteur (SA) de part et d'autre, ceci dans le but de constituer un premier intron synthétique à la suite du premier exon de 20 paires de bases non traduites du gène de la sPLA2-IIA comme représenté schématiquement sur la figure 7A. L'insertion d'un premier intron facilite en général l'expression du trangène situé en aval. Cette insertion permettrait d'une part de restreindre et d'autre part, d'amplifier l'expression du transgène dans les chondrocytes grâce à la présence du site pour le facteur SOX9. Dans une autre construction, la séquence contenant les sites de liaison des facteurs SOX en tant que promoteur peut être insérée avant les sites de liaison des PPARs comme représenté schématiquement sur la figure 7B.

Un autre objet de l'invention concerne tout acide nucléique comprenant un promoteur hybride tel que défini ci-avant et un gène d'intérêt. Le gène d'intérêt peut être tout acide nucléique (ADNc, ADNg, ADN synthétique ou semi-synthétique, ARN, etc.), d'origine diverse (animale, humaine, végétale, bactérienne, virale, artificielle, etc.), codant un produit d'intérêt (ARN, polypeptide ou peptide). Il s'agit avantageusement d'un acide nucléique codant un produit anti-inflammatoire, c'est-à-dire un produit capable de restaurer ou de réduire les phénomènes d'inflammation dans une cellule, tissu ou organe. Parmi les produits anti-inflammatoires, on peut citer notamment le TIMP ou d'autres cytokines ou enzymes capables de réduire le phénomène d'inflammation. Le produit d'intérêt peut être, de manière plus générale, toute enzyme, hormone, facteur de croissance, anticorps, peptide immunogène, lipoprotéine, toxine, anticorps ou fragment d'anticorps, antisens, facteur de transcription, etc., d'intérêt thérapeutique ou vaccinal.

Le gène d'intérêt est généralement placé en aval (3') du promoteur hybride, sous le contrôle transcriptionnel de celui-ci. Ainsi, un acide nucléique préféré au sens de l'invention comprend, dans l'orientation 5'->3' :
a) un élément de réponse à un PPARα et/ou γ, comprenant de préférence tout ou partie d'une séquence SEQ ID NO :1 à 4,
b) tout ou partie de la séquence SEQ ID NO :5,
c) le cas échéant, un élément conférant une spécificité tissulaire, et
d) un gène codant un produit d'intérêt, notamment thérapeutique ou vaccinal.

Un autre objet de l'invention réside dans un vecteur comprenant un promoteur hybride ou un acide nucléique tels que définis ci-avant. Le vecteur peut être de nature et/ou d'origine variées, notamment plasmidique, épisomique, chromosomique, virale, phagique, etc. Préférentiellement, le vecteur est un plasmide ou un virus recombinant, plus préférentiellement un plasmide.

Comme exemple de vecteur viral, on peut citer notamment un adénovirus recombinant, un rétrovirus recombinant, un AAV, un herpès virus, un virus de la vaccine, etc., dont la préparation peut être réalisée selon les méthodes connues de l'homme du métier.

Concernant les plasmides, on peut mentionner tout plasmide réplicatif ou intégratif, compatible avec une utilisation dans les cellules mammifères, notamment humaines.

Un autre objet de l'invention réside dans une composition comprenant un acide nucléique ou un vecteur tel que défini ci-avant. La composition peut contenir par ailleurs tout véhicule acceptable pour une administration in vivo ou ex vivo de ces constructions (notamment tout véhicule pharmaceutiquement acceptable), et/ou pour une conservation stable de celles-ci. Comme véhicule on peut citer notamment des solutions salines, tamponnées, isotoniques, comprenant éventuellement des additifs tels que des polymères ou protéines de haut poids moléculaires. Par ailleurs, les compositions de l'invention peuvent comprendre des agents facilitant la pénétration des constructions génétiques de l'invention dans les cellules. On peut citer notamment des lipides, polymères, peptides, etc., permettant d'améliorer la transfection cellulaire.

A cet effet, une composition particulière selon l'invention comprend un ou plusieurs lipides polycationiques, éventuellement couplés à des polymères polycationiques. Ce type de formulation est particulièrement adapté au transfert de gènes dans les chondrocytes, qui sont entourés par une matrice riche en protéoglycanes chargés négativement. Des lipides polycationiques peuvent donc s'intégrer facilement dans cette matrice. L'adjonction de polymères polycationiques comme le PEI (polyethylèneimine) comprenant des amines incomplètement protonables au pH physiologique permet d'augmenter l'efficacité de la transfection des chondrocytes. Ces amines fonctionnent comme une éponge à protons aux propriétés endosomolytiques. L'ensemble constitué des lipides cationiques, du PEI et du plasmide porteur du transgène constitue une composition particulière préférée dans le cadre de la présente invention.

D'autre part, les compositions de l'invention peuvent aussi comporter un ou plusieurs éléments de ciblage, permettant d'assurer une transfection préférentielle vers des types cellulaires souhaités. Dans ce contexte, les éléments de ciblage peuvent être constitués de ligands de récepteurs spécifiques, de récepteurs de ligands, d'anticorps ou fragments d'anticorps, etc.

Pour un ciblage vers les cellules chondrocytaires, une composition préférée au sens de l'invention comprend en outre une ou plusieurs molécules possédant une affinité pour la membrane chondrocytaire. Parmi ces protéines, on peut mentionner notamment la protéine Chondroadherin, qui interagit avec une bonne affinité avec l'intégrin alpha2 beta1 des chondrocytes (Camper L et al, 1997). Il s'agit d'une petite protéine de 36 kDa riche en leucine. Cette protéine peut être produite (par exemple dans un baculovirus recombinant), purifiée, et incorporée dans une composition (notamment un liposome) de l'invention. L'hydrophobicité de la protéine peut par ailleurs être augmentée par adjonction d'une séquence de farnésylation en C-terminal comme l'extrémité Q-Ras4B (Leevers SJ et al, 1994; Stokoe D et al, 1994; Zlatkine et al, 1997).

Il est entendu que tout autre élément de ciblage peut être envisagé, selon les applications recherchées.

Par ailleurs, un autre objet de l'invention réside encore dans une composition comprenant (i) un acide nucléique ou un vecteur tels que définis ci-avant et (ii) un activateur de PPAR, pour une utilisation simultanée, séparée ou espacée dans le temps.

Comme indiqué ci-avant, les promoteurs hybrides comportent un élément de réponse à un PPAR. Ce PPAR peut être libéré (ou induit) dans les cellules, par exemple en situation d'inflammation, ou également en présence d'activateurs exogènes (i.e., ajoutés ou administrés). Dans ce contexte, l'expression « en vue d'une utilisation simultanée, séparée ou espacée dans le temps » indique que le vecteur et l'activateur peuvent être préparés séparément, conditionnés séparément, et utilisés séquentiellement, pour permettre le contrôle de l'expression de l'acide nucléique d'intérêt. Typiquement, le vecteur et l'activateur sont conditionnés séparément et utilisés de manière espacée dans le temps, la combinaison de ces différents éléments dans une cellule, un tissu, un organe, etc. conduisant à l'effet de régulation ou d'amplification d'expression recherché.

Selon le promoteur hybride ou les applications recherchées, différents types de ligands peuvent être utilisés, naturels ou synthétiques. Ainsi, il peut s'agir préférentiellement d'un activateur de PPARγ et/ou d'un activateur de PPARβ et/ou d'un activateur de PPARα.

Les ligands activateurs des PPARγ peuvent être choisis parmi les ligands naturels et synthétiques. Comme ligands naturels, on peut mentionner les acides gras et les eicosanoïdes (par exemple l'acide linoléique, l'acide linolénique, le 9-HODE, le 5-HODE) et comme ligands synthétiques on peut mentionner les thiazolidinediones, telles que notamment la rosiglitazone (BRL49653), la pioglitazone ou la troglitazone (voir par exemple Krey G. et coli., *Mol. Endocrinol*., 11 (1997) 779-791 ou Kliewer S. et Willson T., *Curr. Opin. in Gen.* Dev. 8 (1998) 576-581), le composé RG12525 ou la 15 dioxy-Δ12-14 PGJ2.

Les ligands activateurs des PPARα sont par exemple les fibrates tels que l'acide fibrique et ses analogues. Comme analogues de l'acide fibrique on peut mentionner notamment le gemfibrozyl (Atherosclerosis 114(1) (1995) 61), le bezafibrate (Hepatology 21 (1995) 1025), le ciprofibrate (BCE&M 9(4) (1995) 825), le clofibrate (Drug Safety 11 (1994) 301), le fénofibrate (Fenofibrate Monograph, Oxford Clinical Communications, 1995), le clinofibrate (Kidney International. 44(6) (1993) 1352), l'acide pirinixique (Wy-14,643), l'acide 5,8,11,14-eicosatetranoique (ETYA), ou les agents anti-inflammatoires non stréroîdiens (AINS) tels que l'ibuprofène et l'indomethacine. Ces différents composés sont compatibles avec une utilisation biologique et/ou pharmacologique in vitro ou in vivo.

Par ailleurs, les compositions selon l'invention peuvent comporter plusieurs activateurs de PPAR en association, et en particulier un fibrate ou un analogue de fibrate associé à un rétinoïde.

Ces différents ligands peuvent être utilisés à des doses conventionnelles, décrites dans l'art antérieur et illustrées dans les exemples.

Les compositions selon l'invention peuvent être formulées dans tout type de matériel adapté, tel que tube, ampoule, poche, fiole, seringue, etc, et conservées au froid (ou congelées) ou sous forme lyophilisée.

L'invention peut être utilisée pour exprimer un gène dans différents types de cellules, de tissus ou d'organes, in vitro, ex vivo. En particulier, il peut s'agir d'une cellule, d'un tissu ou d'un organe mammifère, de préférence humain, en particulier en état d'inflammation. A titre illustratif, on peut citer les cellules chondroytaires (ou de la matrice osseuse) musculaires (ou un muscle), hépatiques (ou le foie), cardiaques (ou le coeur, la paroi artérielle ou vasculaire), nerveuses (ou le cerveau, la moelle, etc) ou tumorales (ou une tumeur).

Préférentiellement, les constructions, compositions et procédé de l'invention sont utilisés pour l'expression régulée d'un acide nucléique dans une cellule chondrocytaire in vitro, ex vivo Les résultats présentés dans les exemples illustrent plus particulièrement les avantages de l'invention in vivo ou in vitro dans ce type de cellules.

L'invention concerne également l'utilisation d'un vecteur tel que défini ci-avant pour la préparation d'une composition destinée à induire l'expression d'un gène dans un tissu en situation d'inflammation. Elle concerne également une composition pour l'expression d'un gène dans un tissu en situation d'inflammation, comprenant un vecteur ou une composition ci-dessus.

Avantageusement, il s'agit d'une composition pour l'expression d'un gène dans un chondrocyte.

L'invention concerne aussi des acides nucléiques, vecteurs ou compositions ci-avant pour le traitement de l'arthrose.

Pour un usage in vitro ou ex vivo, les cellules peuvent être mises en contact avec les compositions ou vecteurs de l'invention selon différents protocoles. Ainsi, les cellules en culture peuvent être incubées directement avec les vecteurs ou compositions de l'invention, par exemple avec un vecteur comportant le promoteur hybride et un gène d'intérêt, le cas échéant en présence de l'activateur. De manière alternative, les cellules peuvent être incubées dans un premier temps avec le vecteur ou acide nucléique puis, dans un deuxième temps (après culture et éventuellement sélection des cellules modifiées), l'activateur peut être ajouté. Ces expériences peuvent être réalisées dans tout dispositif et milieu approprié, de préférence en plaque, boîte, flasque, en condition stérile. Les quantités de cellules, vecteur et ligand peuvent être aisément adaptées par l'homme du métier, sur la base des informations fournies dans les exemples et de ses connaissances générales.

Les cellules (ou organes, tissu, etc.) sont mises en contact par administration des acides nucléiques, vecteurs ou compositions de manière simultanée, séparée ou espacée dans le temps. A cet effet, les vecteurs, compositions ou acides nucléiques sont généralement sous forme administrable par voie parentérale, en particulier intramusculaire, intraveineuse, sous-cutanée, intradermique, intratumorale, intra-osseuse, intra-articulaire ou stéréotaxique. Le choix peut être guidé par l'application envisagée, le tissu ciblé et/ou le type de produit d'intérêt codé par le transgène.

Les compositions de l'invention peuvent comprendre tout agent favorisant la transfection cellulaire (polymère cationique, lipide, etc.).

L'activateur est administrable avant, simultanément, ou après les vecteurs ou acides nucléiques. A cet égard, le ligand est administrable par voie orale, anale, intraveineuse, intrapéritonéale, intraoculaire ou intramusculaire, par exemple.

Les doses utilisées peuvent être adaptées par l'homme du métier, sur la base des données in vivo publiées dans la littérature. Ainsi par exemple, pour une forme non soluble dans l'eau, des doses typiques de ligand tel que BRL 49653 sont comprises entre 5 et 50 mg/kg, par exemple 30 mg/kg, permettant d'obtenir une concentration plasmatique proche de 15µg/ml environ, au moins. Pour une forme hydrosoluble de ligand, dont la biodisponibilité est plus grande (par exemple un sel de maleate du BRL49653) les doses typiques sont plus faibles, généralement inférieures à 5 mg/kg, par exemple de 0,01 à 1 mg/kg. Ces doses peuvent bien évidemment être adaptées par l'homme du métier en fonction des constructions utilisées, des ligands utilisés, et des applications et effets recherchés. Par ailleurs, des administrations répétées de ligand peuvent être réalisées.

De manière générale, les doses de vecteur utilisées peuvent varier entre 0,01 et 1000 µg, ou plus, selon les applications recherchées.

Dans une expérience typique, un vecteur selon l'invention est administrable par voie locale (directement dans le tissu affecté, par exemple le siège de l'inflammation, notamment par voie intra-articulaire) en présence d'un agent de transfection. Sous l'effet des médiateurs libérés par les cellules (PG2, IL1, LTB4, etc), une expression du gène d'intérêt est observée, qui peut être stimulée ou amplifiée par un ou plusieurs ligand des PPAR, tels que définis ci-avant, ou en présence des PPAR endogènes.

Ainsi, la libération d'acide arachidonique et d'autres acides gras par les phospholipases A₂ aboutit à la synthèse d'autres icosanoides et médiateurs lipidiques. Ces médiateurs activent les facteurs de transcription PPARs (Peroxisome Proliferator-Activated Receptors). Ces facteurs sont également activés par certains médicaments comme les fibrates hypolipémiants, les thiazolidinediones utilisées dans le diabète et les médicaments anti-inflammatoires non stéroïdiens (AINS) (Lehmann JM et al, 1997). Les chondrocytes expriment les facteurs PPARα et PPARγ (Bordji et al, J. Biol. Chem., sous presse). Les icosanoïdes (dérivés de l'acide arachidonique) comme les prostaglandines (PGE2, PGJ2) induisent le PPARγ (Kliewer SA et al, 1995; Krey G et al, 1997) ; en particulier la prostaglandine 15 dioxy Δ12-14 PGJ2 est l'activateur le plus puissant de PPARg. Le leucotriène LTB4 et les fibrates activent le PPARα (Devchand RP et al, 1996) et les acides gras longs activent les deux isoformes PPARα et γ.

Les résultats présentés dans les exemples illustrent les propriétés synergiques des promoteurs hybrides, en présence de deux signaux activateurs.

L'invention concerne aussi toute cellule modifiée par mise en contact avec une composition ou un vecteur tels que définis ci-avant, notamment toute cellule mammifère, en particulier humaine, plus spécifiquement de chondrocytes.

L'invention concerne aussi différents fragments du promoteur PLA2s tels que décrits ci-avant, qui sont utilisables par exemple pour conférer un caractère régulable à des promoteurs.

La présente invention sera décrite plus en détails à l'aide des exemples qui suivent qui doivent être considérés comme illustratifs et non limitatifs.

### LEGENDE DES FIGURES

Figure 1 : Représentation schématique d'un promoteur hybride de l'invention contrôlant l'expression du TIMP1.

Figure 2 : Analyse de liaison par retardement sur gel. Les séquences DR1, (DR1)2 17, (DR1)2 21, (DR1)2 31 radiomarquées sont incubées avec 5 ou 10 µl d'extraits Cos-1 enrichis en PPARγ et RXR. Les pistes 1, 4, 7, 10 correspondent à 5µl et les pistes 2, 5, 8, 11 à 10µl d'extraits. Les pistes 3, 6, 9 et 12 correspondent à 10µl d'extraits Cos-1 non transfectées.

Figure 3 : Quantification de la radioactivité obtenue dans les bandes dimériques, tétramériques et la sonde libre (PPARγ).

Figure 4 : Quantification de la radioactivité obtenue dans les bandes dimériques, tétramériques et la sonde libre (PPARα).

Figure 5 : Effet de la PGJ2 (inducteur du PPARγ) et du Wy-14643 (inducteur du PPARα) sur les promoteurs synthétiques.

Figure 6 : Les chondrocytes de lapin ont été transfectés par le plasmide contenant l'élément (DR1)2 21- (-247/+20)sPLA2-CAT. Les différents inducteurs sus-cités sont ajoutés au mileu de culture.

Figures 7A et 7B : Représentation schématique de constructions dans lesquelles des éléments du promoteur du collagène de type Il capables de fixer les facteurs SOX ont été intégrés respectivement en aval et en amont de la construction hybride (DR1)2 21 -promoteur de la sPLA2 IIA.

### DESCRIPTION DETAILLEE DE L'INVENTION

### MATERIELS ET METHODES

### 1-Cultures cellulaires et transfections.

Les pattes antérieures et postérieures de lapines prépubères (Fauve de Bourgogne) de 3 semaines sont disséquées. et découpées dans des conditions stériles. Le cartilage est retiré au scalpel puis les chondrocytes sont dissociés grâce aux enzymes protéolytiques : hyaluronidase 0.05% pendant 15 minutes, Trypsine 0.25 % pendant 30 minutes et collagenase 0.2% pendant 90 minutes. Enfin le milieu HAM's F12 est ajouté pendant 60 minutes. La suspension de chondrocytes est mise en culture dans des boîtes de 6 cm de diamètre (150 000 cellules par boîte) en présence d'un milieu HAM's F12 contenant 10% de sérum de veau foetal. Dans ces conditions, les chondrocytes ont un phénotype différencié comme le démontre la production de collagène de type II.

Quand ces cellules atteignent la pré-confluence (6 à 7 jours), elles sont transfectées avec les différentes constructions en utilisant la méthode de phosphate de calcium.

### 2- Mesure des activités CAT et β-galactosidase.

L'activité CAT est mesurée selon la technique de double phase liquide selon Desbois et al (1992). L'activité β-galactosidase est mesurée dans le but de normaliser les variations de l'efficacité de transfection.

### 3- Préparation des protéines nucléaires et expériences de retard à l'électrophorèse.

Les cellules Cos-1 (fibroblastes de rein de singe transformés) sont transfectées selon la technique de phosphate de calcium sus-citée en utilisant 20 µg des vecteurs d'expression du PPARγ, PPAR α ou le RXR. Des cellules Cos-1 non transfectées serviront de témoin. 48 heures après la transfection les cellules sont incubées avec un tampon contenant du KCl (0.45 M), du Tris-HCl (20 mM, pH=7.5) et 10% de glycérol. Elles sont détachées par grattage, puis cassées par refroidissement à -80°C et décongélation à 37°C. Ces cellules sont ensuite centrifugées à 15000 rpm, pendant 10 minutes et à 4°C. Le surnageant est ensuite congelé à-80°C et utilisé pour les expériences de retardement sur gel.

### 4- Expériences de retardement sur gel.

Les séquences DR1, (DR1)2 17, (DR1)2 21, et (DR1)2 31 sont radiomarquées grâce au fragment Klenow de l'ADN polymérase en utilisant du dCTP α ³²P. Cent mille cpm (0.4 ng) des sondes radiomarquées sont incubés, pendant 30 minutes et à 4°C, avec des quantités croissantes d'extraits Cos-1 enrichis en PPAR ou RXR (comme indiqué dans les légendes des figures) et avec un tampon de liaison spécifique (KCl 100 mM, Hepes 40 mM, pH 7.5, NP40 0.1%, ZnCl₂ 0.5 mM, PEG 8%). Le mélange est ensuite déposé sur un gel d'acrylamide/bisacrylamide (5%, TBE 0.25 X) et migré pendant 2 heures 30 à 200 volts. Le gel est ensuite séché et autoradiographié.

### 5-Biologie Moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose, la purification de fragments d'ADN par électroélution, la précipitation d'ADN plasmidique en milieu salin par l'éthanol ou l'isopropanol, la transformation dans Escherichia coli sont bien connues de l'homme de l'art et sont abondamment décrites dans la littérature (Sambrook et coll., "Molecular Cloning, a Laboratory Manual", Cold Spring Harbor Laboratory, Cold Spring Harbor, N.Y., 1989).

Les plasmides sont d'origine commerciale

L'amplification enzymatique de fragments d'ADN par la technique d'ACP (Amplification en Chaîne par la Polymerase) peut être effectuée en utilisant un DNA thermal cycler™ (Perkin Elmer Cetus) selon les recommandations du fabricant.

L'électroporation d'ADN plasmidique dans des cellules d'Escherichia coli peut être réalisée à l'aide d'un électroporateur (Bio-Rad) selon les recommandations du fabricant.

La vérification des séquences nucléotidiques peut être effectuée par le méthode développée par Sanger et coll. (*Proc. Natl. Acad. Sci. USA,* 74 (1977) 5463-5467) en utilisant le kit distribué par Applied Biosystems selon les recommandations du fabricant.

### EXEMPLES

Dans la partie expérimentale qui suit ont été décrites la construction et l'utilisation de plusieurs promoteurs hybrides de l'invention, comprenant un élément inductible notamment par l'IL1β et un élément inductible par les icosanoïdes, les acides gras et les anti-inflammatoires non-stéroïdiens (AINS).

Le premier module comporte notamment la région [-247; +20] (par rapport au site d'initiation de la transcription de la sPLA2). Cette région contient la boite TATA et l'élément proximal (-114/-85) qui est indispensable à l'activité transcriptionnelle et qui lie le facteur ubiquitaire Sp1. Il comporte également le site C/EBP (-198/-190) indispensable à la stimulation par l'IL-1β et l'élément [-247/-210] qui amplifie l'activité des facteurs C/EBP. de plus, ce module contient un élément de réponse aux glucocorticoïdes capable de relayer une induction par ces hormones.

L'addition du deuxième module vise à amplifier l'induction lors du processus inflammatoire par les médiateurs dérivés des acides gras produits par l'inflammation (prostaglandines, leucotriènes) ou par des composés exogènes (fibrate, médicaments anti-inflammatoires non stéroïdiens-AINS). Ce module comporte un élément de réponse aux PPARs (PPRE) situé en amont du premier module.

### EXEMPLE 1 : Construction d'éléments de réponse aux PPAR

Cet exemple décrit la construction et l'analyse fonctionnelle de plusieurs éléments de réponse aux PPAR, utilisables dans les promoteurs hybrides de l'invention.

### 1-Elaboration d'une unité de réponse aux PPARs de haute affinité

Afin d'obtenir une induction optimale par les ligands du PPAR, plusieurs arrangements de multimères de PPRE ont été imaginés et synthétisés. La séquence des ces régions est donnée ci-après.

L'élément DR1 est le site consensus de liaison de l'hétérodimère PPAR/RXR. Les éléments (DR1)2 17, (DR1)2 21 et (DR1)2 31 sont formés de deux DR1 séparés centre à centre de 17, 21 et 31 paires de bases respectivement.

### 2- Expériences de liaison

### 2-1 liaison de PPARγ

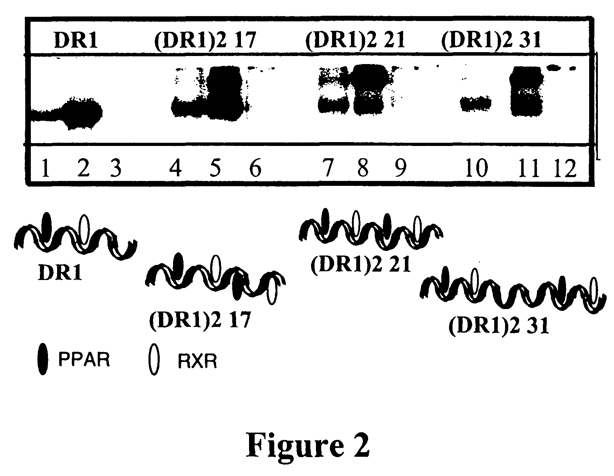

Afin de vérifier le caractère fonctionnel et l'efficacité des régions PPRE ci-dessus, des expériences de retardement sur gel ont été réalisées, consistant à étudier la liaison entre l'une des quatre séquences ci-dessus, préalablement radiomarquée, en présence de quantités croissantes (5 et 10 µl) de protéines PPARγ et RXR préparées dans les cellules Cos-1 (rapport PPARγ/RXR=3 :1). Les résultats obtenus sont présentés sur la Figure 2.

Ces résultats montrent que les séquences construites lient l'hétérodimère PPAR/RXR. La séquence DR1 lie un complexe dimérique alors que les séquences (DR1)2 17, (DR1)2 21 et (DR1) 2 31 lient le complexe dimérique mais aussi un complexe, retardé, de haut poids moléculaire, qui correspondrait à un double hétérodimère PPAR/RXR. Par ailleurs, les quantités des complexes dimérique et de haut poids moléculaire sont variables d'une séquence à l'autre. Ceci suggère que ces trois séquences lient avec une coopérativité différente les complexes RXR/PPARγ.

Afin de savoir si les complexes observés proviennent de la production de PPARγ et de RXR dans les cellules Cos-1, ou bien de protéines présentent dans ces cellules, ces différentes séquences ont été incubées avec des extraits de Cos-1 non transfectées (10µl). L'intensité des complexes formés avec ces extraits est très faible, ce qui est en faveur de la production de protéines PPARγ et RXR suite à la transfection des cellules (Fig. 2).

Dans le but d'étudier la coopérativité de liaison du PPAR/RXR sur ces différentes séquences, des expériences de retardement sur gel en mettant des quantités croissantes d'extraits Cos-1 enrichis en PPAR et RXR (1µl à 10µl), puis de quantification de la radioactivité obtenue, ont été réalisées. La formation du premier dimère PPAR/RXR est caractérisée par la constante de dissociation Kdll, celle du double dimère, ou tetramère, est caractérisée par la constante de dissociation KDIV. Le rapport KdII/KdIV (R) est le reflet de la coopérativité de liaison s'il est supérieur à 1.

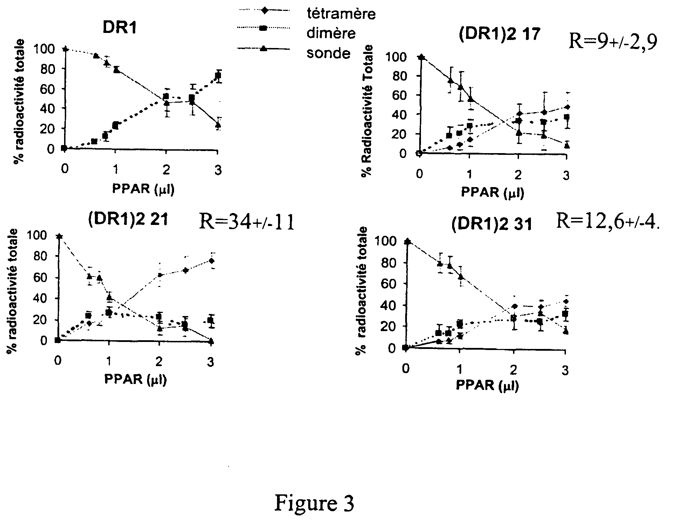

Les différents complexes dimériques, tétramériques et la sonde libre ont été quantifiés grâce à un Phosphorimager et représentés dans la figure 3. Les rapports de coopérativité ont été calculés grâce à la formule sus-citée. Les séquences (DR1)2 17 et (DR1)2 31 lient autant de forme dimérique et tétramériques. Les rapport de coopérativité sont de 9 et 12.6 respectivement. Cependant, la séquence (DR1)2 21 présente un profil de liaison différent. La forme dimérique ne forme que 20% de la liaison totale, alors que la forme tétramérique forme 80% de la liaison totale. Le rapport de coopérativité est de 34 +/- 11.

L'ensemble de ces résultats montre que les trois séquences utilisées lient de manière coopérative le PPAR et le RXR. La séquence qui présente la meilleure coopérativité de liaison est le (DR1)2 21.

### 2-2 liaison de PPARα

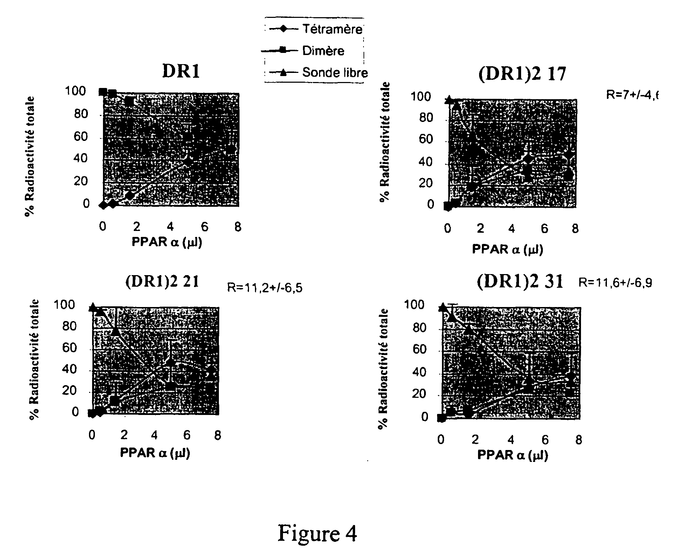

Des expériences similaires ont été réalisées avec le PPARα. Le vecteur d'expression de l'isoforme α du PPAR a été transfecté dans les cellules Cos-1. Des extraits totaux de ces dernières ont été préparés comme décrit dans le chapitre matériels et méthodes. Des expériences de retardement sur gel en utilisant les quatre séquences radiomarquées et des quantités croissantes des extraits Cos-1 enrichis en PPARα et de RXR (rapport3/1) ont été réalisées. Les bandes correspondant aux formes dimériques (D), tétramériques (T) ou la sonde libre (S) ont été découpées du gel puis quantifiées par un comptage sur un compteur à scintillation bêta (Figure 4). Le modèle thermodynamique décrit plus haut a été appliqué dans le but de calculer les rapports de coopérativité de liaison de l'héterodimère PPARα/RXR aux différentes séquences étudiées.

A la différence de ce que qui a été observé avec le PPAR_{γ}, les rapports de coopérativité obtenus avec les séquences (DR1)2 21 et (DR1)2 31 sont équivalents (R=11,2 et 11,6 respectivement). Le rapport de coopérativité obtenu dans le cas de l'élément (DR1)2 17 est inférieur aux deux précédents (R=7). Dans ce cas aussi, les trois séquences synthétisées lient de manière coopérative le PPARα, les séquences (DR)2 21 et (DR1) 2 31 présentant la meilleure coopérativité de liaison.

### EXEMPLE 2 : Construction et analyse fonctionnelle d'un vecteur comprenant un promoteur hybride.

Cet exemple décrit la construction de plasmides comprenant un promoteur hybride de l'invention, et démontre ses propriétés d'induction par différents médiateurs de l'inflammation.

### 1) Construction des plasmides et essais fonctionnels

Le fragment -247/+20 du promoteur de la sPLA2 IIA (SEQ ID NO : 5) a été sous-cloné en amont du vecteur rapporteur CAT du plasmide PUC-SH-CAT. En amont de ce promoteur les différentes séquences PPRE citées ci dessus (SEQ ID NO : 1-4) ont été insérées.

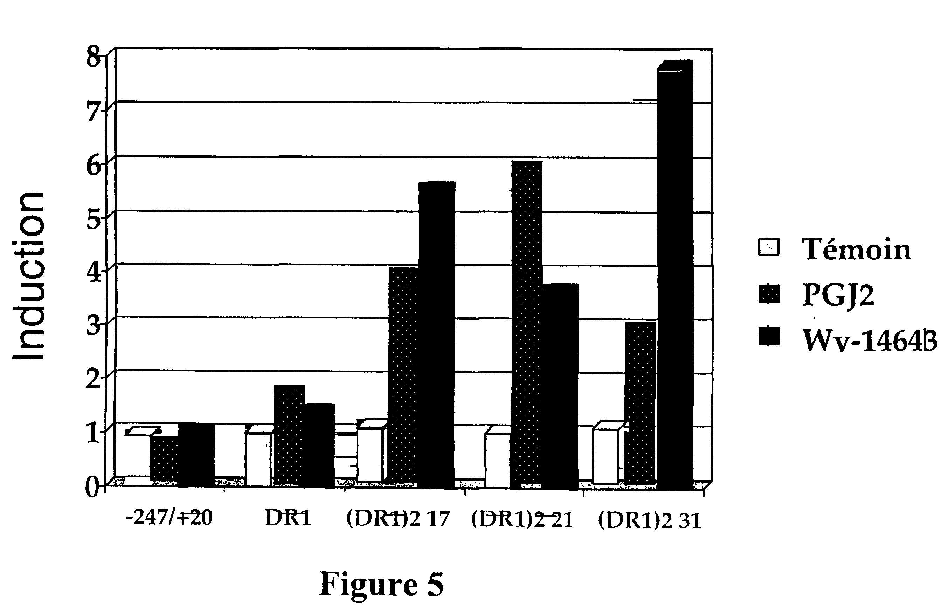

Les chondrocytes de lapin ont ensuite été transfectés par les constructions CAT contenant les différents arrangements de PPRE (DR1) placés en amont du promoteur minimal de la sPLA2-IIA. La 15 deoxy Δ¹²⁻¹⁴ PGJ2 (10µM) (activatrice du PPARγ) ou le Wy-14643 (200 µM) (activateur du PPARα) ont ensuite été ajoutées au milieu de culture pendant 24 heures. Les résultats obtenus sont présentés sur la Figure 5.

Les résultats montrent que le promoteur minimal (-247/+20) de la sPLA2-IIA, qui ne contient pas de sites PPRE connus, n'est pas activé par la PGJ2 ou par le Wy 14643. Cependant, les différents arrangement de DR1, sont activés à différents niveaux par les inducteurs du PPARγ ou du PPARα. On peut les classer dans l'ordre croissant suivant : DR1< (DR1)_{2 17}= (DR1)_{2 31}< (DR1)_{2 21} dans le cas du PPARγ. En comparant les résultats de liaison aux résultats fonctionnels, on observe que la séquence (DR1) 2 21, qui a la meilleure coopérativité de liaison, présente la meilleure synergie fonctionnelle.

Dans le cas du PPAR_{α}, l'ordre d'inductibilité par le Wy-14643 est différent de celui établit dans le cas de la PGJ2. DR1 < (DR1)_{2 21}< (DR1)_{2 17}< (DR1)_{2 31}. Les expériences de retardement sur gel ne sont pas en parfait accord avec les expériences de transfection transitoires. Les expériences de liaison ont montré que les séquences (DR1)_{2 21} et (DR1)_{2 31} lient avec la même coopérative le PPAR_{α} (R=11) alors que la séquence (DR1)_{2 17} présente une coopérativité de liaison moins importante (R=7).

### 2- Effets combinés de l'IL-1, des glucocorticoïdes et des inducteurs de PPARα (fibrates) et PPAR_{γ} (15 dioxy Δ12-14 PGJ2) sur le promoteur synthétique :

Des chondrocytes de lapin ont ensuite été transfectés, soit avec le promoteur minimal de la sPLA2-IIA, soit avec la séquence (DR1)_{2 21} placée en amont de ce dernier (promoteur hybride). La PGJ2 (10µM) et/ou l'IL-1 (10ng/ml) sont rajoutées dans le milieu de culture.

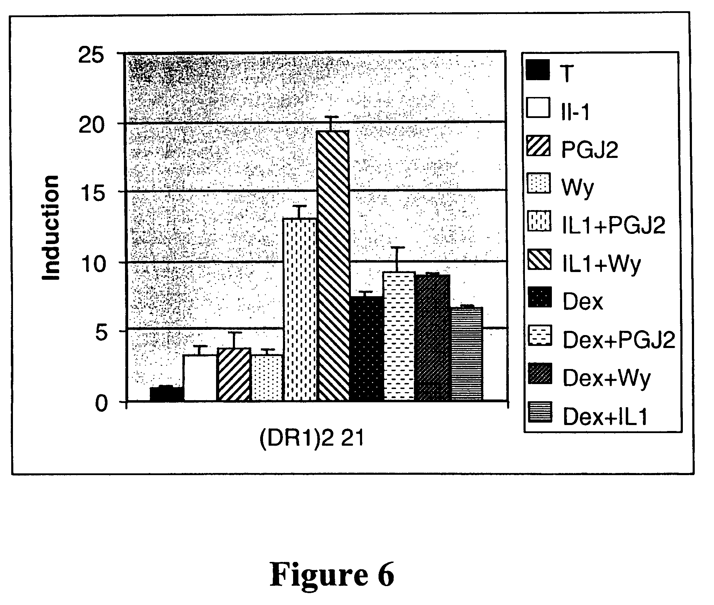

Les résultats obtenus montrent que l'IL-1 induit le promoteur de la sPLA2-IIA d'environ 6 fois. La prostaglandine J2 ou le Wy-14643 n'ont pas d'effet sur ce promoteur. Dans le cas de la construction (DR)2 21-(-247/+20) sPLA2-CAT, L'IL-1 et la PGJ2 induisent ce promoteur de 4 fois. Les deux produits combinés ont un effet synergique (induction par rapport à l'activité basale de 13 fois) (Fig 6).

Ce même type d'expérience a été réalisé avec le Wy-14643. Ce composé est un inducteur spécifique du PPARα. Le Wy-14643 (200 µM) active la transcription de la construction (DR)2 21-(-247/+20) sPLA2-CAT d'environ 3 à 4 fois. L'effet de l'IL-1 et du Wy-14643 combinés est synergique (induction de 20 fois).

L'effet de la dexaméthasone a par ailleurs été testé sur le promoteur synthétique (-247/+20)-(DR1)2 21-CAT, soit seule soit combinée à du Wy-16463, de la PGJ2, ou de l'IL-1. La dexaméthasone active la transcription de ce promoteur d'environ 6 fois. Ces résultats montrent que les glucocorticoïdes circulants comme le cortisol, ou bien administrés lors d'un traitement peuvent activer ce promoteur hybride.

L'effet de certains médicaments anti-inflammatoires non stéroïdiens comme l'indométhacine ou l'ibuprofène a également été testé sur les promoteurs synthétiques. Les résultats obtenus montrent que l'indométhacine (10⁻⁴ M) active d'environ cinq fois la contruction (DR1)2 21-(-247/+20)-CAT alors que l'ibuprofène (10⁻⁴ M) active de 17 fois ce promoteur.

Enfin, les niveaux d'activités transcriptionnelles atteints par un promoteur viral, comme le promoteur RSV (Rous Sarcoma Virus), et celui du promoteur synthétique ont été comparés. L'activité transcriptionnelle basale du promoteur synthétique est faible, elle est inférieure à 5% de l'activité du promoteur viral RSV. Après induction par les différents effecteur, le promoteur synthétique présente un niveau d'activité transcriptionnelle qui atteint 40% de celui de RSV (résultats non montrés). Le promoteur construit selon la présente invention est donc caractérisé par une activité transcriptionnelle induite forte comparable aux vecteurs viraux utilisés actuellement en thérapie génique, et une activité basale faible.

### Conclusions

Des promoteurs synthétiques hybrides inductibles par les composantes de l'inflammation, c'est à dire par les cytokines pro-inflammatoires comme l'IL-1 β, ainsi que par les acides gras pro-inflammatoires, comme les prostaglandines ou les leucotriènes ont été mis au point. De plus, les glucocorticoïdes, circulants ou administrés localement, ainsi que les médicaments anti-inflammatoires non stéroïdiens, comme l'ibuprofène ou l'indométhacine, sont capables d'activer la transcription de ces promoteurs. Ces promoteurs constituent donc un moyen efficace de moduler l'activité de gènes en fonction de la prise ou non d'un traitement anti-inflammatoire. Ces caractéristiques permettent d'envisager un effet controlable (on/off) sur la synthèse du gène-médicament. Il est important de noter, que ce promoteur synthétique présente une activité basale très faible, alors qu'il a une activité transcriptionnelle induite par les stimuli endogènes ou exogènes d'une efficacité comparable aux vecteurs viraux..

### REFERENCES BIBLIOGRAPHIQUES

*Amin AR, Attur M, Patel RN, Thakker GD, Marshall PJ, Rediske J, Stuchin SA, Patel IR, Abramson SB (1997) Superinduction of cyclooxygenase-2 activity in human osteoarthritis-affected cartilage. Influence of nitric oxide. J Clin Invest 99 (6): 1231-1237.*

*Berenbaum F, Thomas G, Poiraudeau S, Bereziat G, Corvol MT, Masliah J (1994) Insulin-like growth factors counteract the effect of interleukin 1 beta on type II phospholipase A*_{*2*} *expression and arachidonic acid release by rabbit articular chondrocytes. FEBS Lett 340 (1-2): 51-55.*

*Bingham C. O. 3*^{*rd*}*, Murakami M., Fujishima H., Hunt J. E., Austen K. F., Arm J. P. (1996) A heparin-sensitive phospholipase A*_{*2*} *and prosaglandin endoperoxide synthase-2 are functionally linked in the delayed phase of prostaglandin D2 generation in mouse bone marrow-derived mast cellls. J. Biol. Chem. 271: 25936-25944.*

*Camper L, Heinegard D, Lundgren-Akerlund E (1997) Integrin alpha2betal is a receptor for the cartilage matrix protein chondroadherin. J Cell Biol ;138(5):1159-6*

*Chevalier, X. Physiopathologie de l'arthrose. (1998) La Presse Médicale. 27(2). 75-92.*

*Clark JD, Lin LL, Kriz RW, Ramesha CS, Sultzman LA, Lin AY, Milona N, Knopf J (1991) A novel arachidonic acid-selective cytosolic PLA*_{*2*} *contains a Ca*^{*2+*} *dependent translocation domain with homology to PKC and GAP. Cell 65: 1043-1051.*

*Dean, J, Martel-Pelletier, J, Pelletier, JP, et al. Evidence for metalloprotease and metalloprotease inhibitors imbalance in human osteoarthritic cartillage. Arthritis Rheum. 1990, 33, 1466-76*

*Desbois C, Massé T, Madjar JJ. (1992) Optimization of the CAT assay procedure by determining the initial rate of the enzymatic reaction. Trends Genet; 8 : 300-30*

*Devchand, RP, Keller, H, Peters, JM, Vazquez, M, Gonzalez, FJ, Wahli, W. The PPAR* ?-*leukotriene B4 pathway to inflammation control. (1996) Nature, 384, 39-43.*

*Di Battista JA, Dore S, Martel-Pelletier JP (1996) Prostaglandin E2 stimulates incorporation of proline into collagenase digestible proteins in human articular chondrocytes: identification* *of an effector autocrine loop involving insulin-like growth factor I*. *Mol Cell Endocrinol 123: 27-35.*

*Geng Y, Blanco FJ, Cornelisson M, Lotz M (1995) Regulation of cyclooxygenase-2 expression in normal human articular chondrocytes. J Immunol 155 (2): 796-801*

*Guingamp C, Gegout-Pottie, Philippe L, Terlain B, Netter P, Gillet P. (1997) Mono-iodoacetate-induced experimental osteoarthriris. Arthritis Rhumatism. 40:1670-1679. Hornebeck W, Lafuma, C. Les métalloproléinases matricielles. (1990) C R Soc biol. 185, 1466-76*

*Hulkower KI, Hope WC, Chen T, Anderson CM, Coffey JW, Morgan DM (1992) Interleukin-1? stimulates cytosolic phospholipase A*_{*2*} *in rheumatoid synovial fibroblasts. Biochem. Biophys. Res. Commun. 184: 712-718.*

*Jacques, C., Bereziat, G., Humbert, L., Olivier, J. L., Corvol, M. T., Masliah, J., and Berenbaum, F. (1997). Posttranscriptional effect of insulin-like growth factor-I on interleukin-1beta-induced type II-secreted phospholipase A2 gene expression in rabbit articular chondrocytes. J Clin Invest 99, 1864-72.*

*Jpenberg AI, Jeannin E, Wahli W, Desvergne B. (1997) Polarity and specifie sequence requirements of peroxisome proliferator-activated receptor (PPAR)*/*retinoid X receptor heterodimer binding to DNA.*

*Kim Y, Fischer SM (1998)Transcriptional regulation of cyclooxygenase-2 in mouse skin carcinoma cells. Regulatory role of CCAAT*/*enhancer-binding proteins in the differential expression of cyclooxygenase-2 in normal and neoplastic tissues. J Biol Chem 16; 273(42):27686-9*

*Klapisz E, Ziari M, Wendum L, Koumanov K, Brachet C,-Ducos, Olivier JL, Béréziat G, Trugnan G, Masliah J (1999) N- and C-terminal plasma membrane anchoring modulate differently agonist-induced activation of cytosolic phospholipase A2 (publication en cours).*

*Kliewer, SA, Lenhard, JM, Wilson, TM, Patel, I, Morris, DC, Lehmann, JM. A prostaglandine J2 metabolite binds peroxisome proliferator-activated receptor ? and promotes adipocyte differentiation. (1995) Cell. 83, 813-819*

*Knott I, Dieu M, Burton M, Houbion A, Remacle J, Raes M (1994) Induction of cyclooxygenase by interleukin l: comparative study between human synovial cells and chondrocytes. J Rheumatol 21 (3): 462-466.*

*Kreiss P, Scherman D (1999) Optimisation des plasmides et des vecteurs synthétiques pour la thérapie génique. Médecine*/*Sciences. 15 : 669-676*

*Krey, G, Braissant, O, L'Horset, F, Kalkhoven, E, Perroud, M, Parker, MG, Wahli. W. Fatty acids, eicosanoids, and hypolipidemic agents identified as ligands of peroxisome proliferator-activated receptors by coactivator-dependent receptor ligand assay. (1997) Mol. Endocrinol. 11, 779-791.*

*Kuwata H., Nakatani Y., Murakami M. & Kudo I. (1998) Cytosolic phospholipase A*_{*2*} *is required for cytokine-induced expression of type IIA secretory phospholipase A*_{*2*} *that mediates optimal cyclooxygenase-2-dependent delayed prostaglandin E2 generation in rat 3Y1 fibroblasts. J. Biol. Chem. 273: 1733-40.*

*Leevers SJ, Paterson HF, Marshall CJ (1994) Requirement for Ras in Raf activation is overcome by targeting Raf to the plasma membrane. Nature 369, 411-414.*

*Lefebvre, V & de Crombrugghe, B. Toward understanding SOX9 function in chondrocyte differentiation. Matrix Biol. 16, 529-540*

*Lefebvre, V, Huang, W, Harley, VR, Goodfellow, PN & de Crombrugghe, B. SOX9 is a potent activator of the chondrocyte-specific enhancer of the pro alpha l (II) collagen gene. (1997) Mol. Cell. Biol. 17, 2336-2346*

*Lehmann JM, Lenhard JM, Oliver BB, Gordan MR, Kliewer SA. (1997). Peroxisome Proliferator-activated receptord a and are ectivated by indomethacin and other non-steroidal anti-inflammatory drugs. J. Biol. Chem. 272:3406-3410.*

*Lin M. K., Farewell V., Vadas P., Bookman A. A., Keystone E. C., Pruzanski W. (1996) Secretory phospholipase A2 as an index of disease in rheumatoid arthritis. Prospective double blind study of 212 patients. J. Rheumatol. 23: 112-1166.*

*Makihira S, Yan W, Ohno S, Kawamoto T, Fujimoto K, Okimura A, Yoshida E, Noshiro M, Hamada T, Kato Y. Enhancement of cell adhesion and spreading by a cartilage-specific* *noncollagenous protein, cartilage matrix protein (CMP*/*Matrilin-1), via integrin alphalbetal. (1999) JBiol Chem , 274(16):11417-2*

*Mukhopadhyay, K, Lefebvre V, Zhou, G, Garofalo, S, Kimura, JH and de Crombrügghe, B. Use of a new rat chondrosarcoma cell line to delineate a 119-base pair chondrocyte-specific enhancer element and to define active promoter segments in the mouse pro-alpha1 (II) collagen gene (1995) J. Biol. Chem. 270, 27711-19.*

*Nevalainen TJ, Marki F, Kortesuo PT, Grutter MG, Di Marco S. Schmitz A (1993) Synovial type (group II) phospholipase A*_{*2*} *in cartilage. J Rheumatol 20 (2): 325-330.*

*Pruzanski W., Albin-Cook K., Laxer R. M., MacMillan J., Stefanski E., Vadas P., and Silverman E. D. (1994) Phospholipase A*_{*2*} *in juvenile rheumatoid arthritis: Correlation to disease type and activity. J. Rheumatol. 21: 1951-1954.*

*Schoonjans, K, Staels, B, Auwrex, J. The peroxisome proliferator activated receptors (PPARs) and their effects on lipid metabolism and adipocyte differentiation. (1996) Biochem. Biophys. Acta. 1302, 93-109.*

*Seilhamer JJ, Pruzanski W, Vadas P., Plant S, Miller JA, Kloss J, Johnson LK (1989) Cloning and recombinant expression of phospholipase A*_{*2*} *present in rheumatoid arthritic synovial fluid. J. Biol. Chem. 264: 5335-5338.*

*Stokoe D, Macdonald SG, Cadwallader K, Symons M, Hancock JF (1994) Activation of Raf as a result of recruitment to the plasma membrane. Science 264, 1463-1467.*

*Thomas B, Humbert L, Crofford L, Biu X, Berenbaum F, Olivier JL. Critical role of C*/*EBP*δ *and C*/*EBPβ factors in the stimulation of cyclooxygenase-2 gene transcription by interleukine-1β in primary culture chondrocyte (publication en cours).*

*Zhou, G, Garofalo, S, Mukhopadhyay, K, Lefebvre, V, Smith, CN, Eberspaecher, H and de Crombrügge, B. A 182 bp fragment of the mouse alpha 1 (II) collagen gene is sufficient to direct chondrocyte expression in transgenic mice (1995) J. Cell. Sci. 108, 3677-84.*

*Zlatkine P, Mehul B, Magee AI (1997) Retargeting of cytosolic proteins to the plasma membrane by the Lck protein tyrosine kinase dual acylation motif. J. Cell. Science 110, 673-679.*

## Revendications

1. Promoteur hybride comprenant:
a) un élément de réponse à un peroxisome proliferator activated receptor PPAR comprenant un ou plusieurs sites de liaison du PPAR et
b) tout du promoteur du gène humain de la Phospholipase A2 sécrétée non-pancréatique PLA2s ou partie de celui-ci assurant une activité transcriptionnelle basale au promoteur hybride et un caractère régulable par les médiateurs de l'inflammation dans lequel l'élément (a) est positionné en amont de l'élément (b).

2. Promoteur selon la revendication 1, **caractérisé en ce que** l'élément (a) de réponse à un PPAR comprend un ou ptusieurs sites de séquence SEQ ID NO : 1 ou de variants fonctionnels de cette séquence.

3. Promoteur selon la revendication 2, **caractérisé en ce que** l'élément (a) de réponse à un PPAR comprend la séquence SEQ ID NO : 1, 2, 3 ou 4 ou un variant fonctionnel de celles-ci.

4. Promoteur selon l'une quelconque des revendications précédentes, **caractérisé en ce que** l'élément b) comprend tout ou partie de la séquence SEQ ID NO : 5.

5. Promoteur selon l'une quelconque des revendications précédentes, **caractérisé en ce que** l'élément b) comprend au moins une partie du promoteur PLA2S conférant l'induction par l'interleukine-1β.

6. Promoteur selon la revendication 4 ou 5, **caractérisé en ce que** l'élément (b) comprend au moins les résidus 51 à 61 de SEQ ID NO:5.

7. Promoteur selon la revendication 4 ou 5, **caractérisé en ce que** l'élément (b) comprend au moins les résidus 5 à 170 de SEQ ID NO : 5.

8. Promoteur selon la revendication 4 ou 5, **caractérisé en ce que** l'élément (b) comprend au moins les résidus 51 à 170 de SEQ ID No : 5.

9. Promoteur selon l'une quelconque des revendications précédentes, **caractérisé en ce que** l'élément a) est positionné en amont de l'élément (b).

10. Promoteur selon l'une quelconque des revendications précédentes, **caractérisé en ce qu'**il comporte en outre un élément c) conférant une spécificité tissulaire.

11. Promoteur selon la revendication 10, **caractérisé en ce que** l'élément c) confère une spécificité pour les cellules chondrocytaires, et notamment **en ce qu'**il comprend tout ou partie de la séquence SEQ ID NO : 7 ou d'un variant de celle-ci.

12. Acide nucléique comprenant un promoteur hybride selon l'une des revendications 1 à 11 et un gène d'intérêt.

13. Acide nucléique selon la revendication 12, **caractérisé en ce que** le gène d'intérêt est un gène codant pour un produit anti-inflammatoire.

14. Vecteur comprenant un promoteur hybride selon l'une des revendications 1 à 11 ou un acide nucléique selon l'une des revendications 12 ou 13.

15. Vecteur selon la revendication 14, **caractérisé en ce qu'**il s'agit d'un plasmide.

16. Utilisation de l'acide nucléique selon la revendication 12 ou 13 ou du vecteur selon la revendication 14 ou 15 pour la préparation d'une composition utile pour le traitement de l'arthrose.

## Patentansprüche

1. Hybrider Promotor umfassend:
(a) ein Peroxisom Proliferator Activated Receptor (PPAR) Response-Eiement umfassend eine oder mehrere Bindungsstellen des PPAR und
(b) den gesamten Promotor des menschlichen Gens der sekretierten, nichtpankreatischen Phospholipase A2 (PLA2s) oder einen Teil desselben,
wobei eine basale Transkriptionsaktivität des hybriden Promotors und ein regulierbarer Charakter durch die Entzündungsvermittler sichergestellt wird,
wobei das Element (a) stromaufwärts des Elements (b) positioniert ist.

2. Promotor nach Anspruch 1, **dadurch gekennzeichnet, dass** das PPAR-Response-Element (a) einen oder mehrere Bereiche der Sequenz SEQ ID NO: 1 oder funktionelle Varianten dieser Sequenz umfasst.

3. Promotor nach Anspruch 2, **dadurch gekennzeichnet, dass** das PPAR Response-Element (a) die Sequenz 1, 2, 3 oder 4 oder eine funktionelle Variante derselben umfasst.

4. Promotor nach einem der vorhergehenden Ansprüche, **dadurch gekennzeichnet, dass** das Element (b) die gesamte oder einen Teil der Sequenz SEQ ID NO: 5 umfasst.

5. Promotor nach einem der vorhergehenden Ansprüche, **dadurch gekennzeichnet, dass** das Element (b) mindestens einen Teil des PLA2S Promotors umfasst, welcher die Induktion durch Interleukin-1 β vermittelt.

6. Promotor nach Anspruch 4 oder 5, **dadurch gekennzeichnet, dass** das Element (b) mindestens die Reste 51 bis 61 der SEQ ID NO: 5 umfasst.

7. Promotor nach Anspruch 4 oder 5, **dadurch gekennzeichnet, dass** das Element (b) mindestens die Reste 5 bis 170 der SEQ ID NO: 5 umfasst.

8. Promotor nach Anspruch 4 oder 5, **dadurch gekennzeichnet, dass** das Element (b) mindestens die Reste 51 bis 170 der SEQ ID NO: 5 umfasst.

9. Promotor nach einem der vorhergehenden Ansprüche, **dadurch gekennzeichnet, dass** das Element (a) stromaufwärts des Elements (b) positioniert ist.

10. Promotor nach einem der vorhergehenden Ansprüche, **dadurch gekennzeichnet, dass** er außerdem ein Element (c) enthält, welches eine Gewebespezifität vermittelt.

11. Promotor nach Anspruch 10, **dadurch gekennzeichnet, dass** das Element (c) eine Spezifität für Chondrozyten vermittelt, insbesondere **dadurch**, dass er die gesamte oder einen Teil der Sequenz SEQ ID NO: 7 oder eine Variante derselben umfasst.

12. Nucleinsäure umfassend einen hybriden Promotor nach einem der Ansprüche 1 bis 11 und ein Gen von Interesse.

13. Nucleinsäure nach Anspruch 12, **dadurch gekennzeichnet, dass** das Gen von Interesse ein Gen ist, welches für ein anti-inflammatorisches Produkt codiert.

14. Vektor umfassend einen hybriden Promotor nach einem der Ansprüche 1 bis 11 oder eine Nucleinsäure nach einem der Ansprüche 12 oder 13.

15. Vektor nach Anspruch 14, **dadurch gekennzeichnet, dass** es sich um ein Plasmid handelt.

16. Verwendung der Nucleinsäure nach einem der Ansprüche 12 oder 13 oder des Vektors nach Anspruch 14 oder 15 zur Herstellung einer Zusammensetzung, welche nützlich zur Behandlung der Arthrose ist.

## Claims

1. Hybrid promoter comprising:
a) a Peroxisome Proliferator Activated Receptor (PPAR response element) comprising one or more PPAR biding sites and
b) the whole promoter of the human gene for non-pancreatic secreted A2 phospholipase PLA2S or part of it ensuring a basal transcriptional activity to the hybrid promoter and the regulation through the mediators of inflammation, in which element (a) is positioned upstream of element (b).

2. Promoter according to claim 1, **characterized in that** the PPAR response element (a) comprises one or more sites having the sequence SEQ ID NO:1 or functional variants of this sequence.

3. Promoter according to claim 2, **characterized in that** the PPAR response element (a) comprises the sequence SEQ ID NO1, 2, 3 or 4 or a functional variant thereof.

4. Promoter according to any one of the preceding claims, **characterized in that** element b) comprises the whole or part of the sequence SEQ ID NO: 5.

5. Promoter according to any one of the preceding claims, **characterized in that** element b) comprises at least part of the PLA2s promoter conferring induction by interleukin-1β.

6. Promoter according to claim 4 or 5 , **characterized in that** element (b) comprises at least residues 51 to 61 of SEQ ID NO :5.

7. Promoter according to claim 4 or 5, **characterized in that** element (b) comprises at least residues 5 to 170 of SEQ ID NO :5.

8. Promoter according to claim 4 or 5, **characterized in that** element (b) comprises at least residues 51 to 170 of SEQ ID NO :5.

9. Promoter according to any one of the preceding claims, **characterized in that** element a) is positioned upstream of element (b).

10. Promoter according to any one of the preceding claims, **characterized in that** it comprises, in addition, an element c) conferring tissue specificity.

11. Promoter according to claim 10, **characterized in that** element c) confers specificity for the chondrocytic cells, and in particular **in that** it comprises the whole or part of the sequence SEQ ID NO:7 or a variant thereof.

12. Nucleic acid comprising a hybrid promoter according to one of claims 1 to 11 and a gene of interest.

13. Nucleic acid according to claim 12, **characterized in that** the gene of interest is a gene encoding an anti-inflammatory product.

14. Vector comprising a hybrid promoter according to one of claims 1 to 11 or a nucleic acid according to either of claims 12 or 13.

15. Vector according to claim 14, **characterized in that** it is a plasmid.

16. Use of a nucleic acid according to either of claims 12 and 1 or a vector according to either of claims 14 and 15 for the preparation of a composition for osteoarthritis treatment.


### LISTE DE SEQUENCES

<110> AVENTIS PHARMA S.A.
<120> PROMOTEURS HYBRIDES
<130> PPAR INFLAMMATION
<140>
   <141>
<160> 7
<170> PatentIn Ver. 2.1
<210> 1
   <211> 20
   <212> ADN
   <213> Séquence artificielle
<220>
   <223> Description de la séquence artificielle:élément PPRE
<400> 1
   caaaactagg tcaaaggtca 20
<210> 2
   <211> 38
   <212> ADN
   <213> Séquence artificielle
<220>
   <223> Description de la séquence artificielle:élément PPRE
<400> 2
   caaaactagg tcaaaggtca aaactaggtc aaaggtca 38
<210> 3
   <211> 41
   <212> ADN
   <213> Séquence artificielle
<220>
   <223> Description de la séquence artificielle:élément
<400> 3
   caaaactagg tcaaaggtca tcaaaactag gtcaaaggtc a 41
<210> 4
   <211> 52
   <212> ADN
   <213> Séquence artificielle
<220>
   <223> Description de la séquence artificielle:élément PPRE
<400> 4
   caaaactagg tcaaaggtca tgtctttagg cccaaaacta ggtcaaaggt ca 52
<210> 5
   <211> 271
   <212> ADN
   <213> Séquence artificielle
<220>
   <223> Description de la séquence artificielle:fragment du promoteur PLA2s
<400> 5
   cgcggcaaaa ctgcctgaaa tgtgttttgg catcagctac tgacacgtaa ggtttcccaa 60
   tcctcaactc tgtcctgcca gctgatgagg ggaaggaaag ggattaccta ggggtatggg 120
   cgaccaatcc tgagtccacc aactgaccac gcccatcccc agccttgtgc ctcacctacc 180
   cccaacctcc cagagggagc agctatttaa ggggagcagg agtgcagaac aaacaagacg 240
   gcctggggat acaactctgg agtcctctga g                                271
<210> 6
   <211> 332
   <212> ADN
   <213> Séquence artificielle
<220>
   <223> Description de la séquence artificielle/ promoteur hybride PPRE/PLA2s
<400> 6
   gtaccaattc gacaaaacta ggtcaaaggt catcaaaact aggtcaaagg tcaaattcga 60
   acgcggcaaa actgcctgaa atgtgttttg gcatcagcta ctgacacgta aggtttccca 120
   atcctcaact ctgtcctgcc agctgatgag gggaaggaaa gggattacct aggggtatgg 180
   gcgaccaatc ctgagtccac caactgacca cgcccatccc cagccttgtg cctcacctac 240
   ccccaacctc ccagagggag cagctattta aggggagcag gagtgcagaa caaacaagac 300
   ggcctgggga tacaactctg gagtcctctg ag                               332
<210> 7
   <211> 944
   <212> ADN
   <213> Séquence artificielle
<220>
   <223> Description de la séquence artificielle:séquence conférant la spécificité d'expression
<400> 7
   tgccggcctc gcggtgagcc ctgatccgcc tcggggctcc ccagtcgctg gtgctgctga 60
   cgctgctcat cgccgcggtc ctacggtgtc agggccagga tgcccgtaag tcgcccgccg 120
   cccctgccta cttccctgac ttgtgaccct tttcctccta ctccctcccc caagtactag 180
   gatcccccta gagcttgcag atctgggatt ggcagcgatg gcttccagat gggctgaaac 240
   cctgcccgta tttatttaaa ctggttcctc gtggagagct gtgaatcggg ctctgtatgc 300
   gcttgagaaa agccccattc atgagaggca aggcccagtg ggtcccccaa ctccccgacc 360
   cccctctccc acaatgcaca gcctccccgc cctcatcccc cccccacccc ccgtgcccgc 420
   ctgccgccac cttcagatcg atctgggatt ggcagcgatg gcttccagat gggctgaaac 480
   cctgcccgta tttatttaaa ctggttcctc gtggagagct gtgaatcggg ctctgtatgc 540
   gcttgagaaa agccccattc atgagaggca aggcccagtg ggtcccccaa ctccccgacc 600
   cccctctccc acaatgcaca gcctccccgc cctcatcccc ccccccaccc cccgtgcccg 660
   cctgccgcca cctccagatc tccagctaga ggatctgcga ctctagggtt cgaaatcgat 720
   aagccaagct ctagtggatc ccccgggctg cagatctgta gggcgcagta gtccagggtt 780
   tccttgatga tgtcatactt atcctgtccc ttttttttcc acagctcgcg gttgaggaca 840
   aactcttcgc ggtctttcca gtggggatcg acggtatcga taagcttgat gatctgtgac 900
   atggcggatc ccgtcgtttt acaacgtcgt gactgggaaa accc                 944